Europäisches Patentamt

European Patent Office

Office européen des brevets

(11) Publication number: **0 194 446**
A1

(12) **EUROPEAN PATENT APPLICATION**

(21) Application number: **86101545.1**

(22) Date of filing: **17.10.83**

(51) Int. Cl.⁴: **A 61 F 7/10**
**A 61 F 7/00**

(30) Priority: **25.10.82 JP 162214/82**

(43) Date of publication of application:
**17.09.86 Bulletin 86/38**

(84) Designated Contracting States:
**AT BE DE FR IT NL SE**

(60) Publication number of the earlier application
in accordance with Art. 76 EPC: **0 107 949**

(71) Applicant: **JUNKOSHA CO. LTD.**
**25-25, Miyasaka 2-chome**
**Setagaya-ku Tokyo156(JP)**

(72) Inventor: **Suzuki, Hirosuke**
**21-8 Kotesashi-Machi 4-Chome**
**Tokorocawa-Shi Saitama(JP)**

(72) Inventor: **Kobayashi, Satoru**
**927-3 Aka-Nakacawa Ohaza-Takahagi**
**Hitaka-Machi Iruma-Gun Saitama(JP)**

(74) Representative: **Taylor, Derek George et al,**
**Mathisen, Macara & Co. The Coach House 6-8 Swakeleys**
**Road**
**Ickenham Uxbridge UB10 8BZ(GB)**

(54) **A cooler for human tissue for use during hyperthermia treatment against cancer.**

(57) A cooling device is provided for use in cooling surrounding human tissue during hyperthermia treatment of cancerous tumour. The device comprises at least one tube (8), wound in a flat coil of closely adjacent turns, constructed of a fluoreresin having continuous pores, such as porous, expanded poltetrafluoroethylene, and through which flows a coolant. Microwaves used in hyperthermia treatment of cancerous tumours can pass through the device and heat the turmour to be treated while the adjacent noncancerous tissue is cooled by the coolant. Because the tube is porous, the cooling effect is enhanced.

_Fig.2._

EP 0 194 446 A1

## A COOLER FOR HUMAN TISSUE FOR USE DURING HYPERTHERMIA TREATMENT AGAINST CANCER

The present invention relates to a apparatus for use in the heat treatment of cancerous tumours. In such treatment, called hyperthermic treatment, a cancerous region of a human is heated, in particular to 41-43°C, with heat producing microwave energy to destroy the cancerous cells.

In conventional heat therapy apparatus of this kind a heater, in the form of a microwave antenna, is disposed on human epithelial tissue to heat the cancerous region which may be located deep in the human body.

Unfortunately, in such therapy, the density of electromagnetic waves is highest just below the antenna and the temperature in the cancerous region should be kept constant. Therefore, the epithelial tissue may be higher in temperature than the cancerous region, and cooling of the epithelial tissue is required.

It is known from US-A-4,140,130 and WO-A-8 001 462 to provide apparatus for use in the treatment of cancerous tumours which compises a microwave heater, the heater incorporating a tube through which refrigerant can be passed.

According to the present invention there is provided apparatus for use in cooling the tissue surrounding a cancerous tumour in a patient undergoing heat treatment of the cancerous tumour by microwave energy characterised by at least one tube wound in a flat coil of closely adjacent turns through which a refrigerant liquid can be caused to flow, the tube being made of a porous, expanded polytetrafluoroethylene resin having interconnecting pores.

The invention will now be particularly described with reference to the accompanying drawings in which:-

Fig. 1 is a diagrammatic view of prior art apparatus for the heat treatment of cancerous tumours;

Fig. 2 is a diagrammatic view of heat treatment apparatus incorporating the present invention;

Fig. 3 is a perspective view of a cooling device according to the invention;

Fig. 4 is a fragmentary plan view of another cooling device according to the invention;

Fig. 4A is a fragmental side elevation of the tube used in the invention showing inlet/outlet ports.

Fig. 5 is a top plan view of a further embodiment of the invention; and

Fig. 6 is a cross-sectional view through the device of Fig. 5.

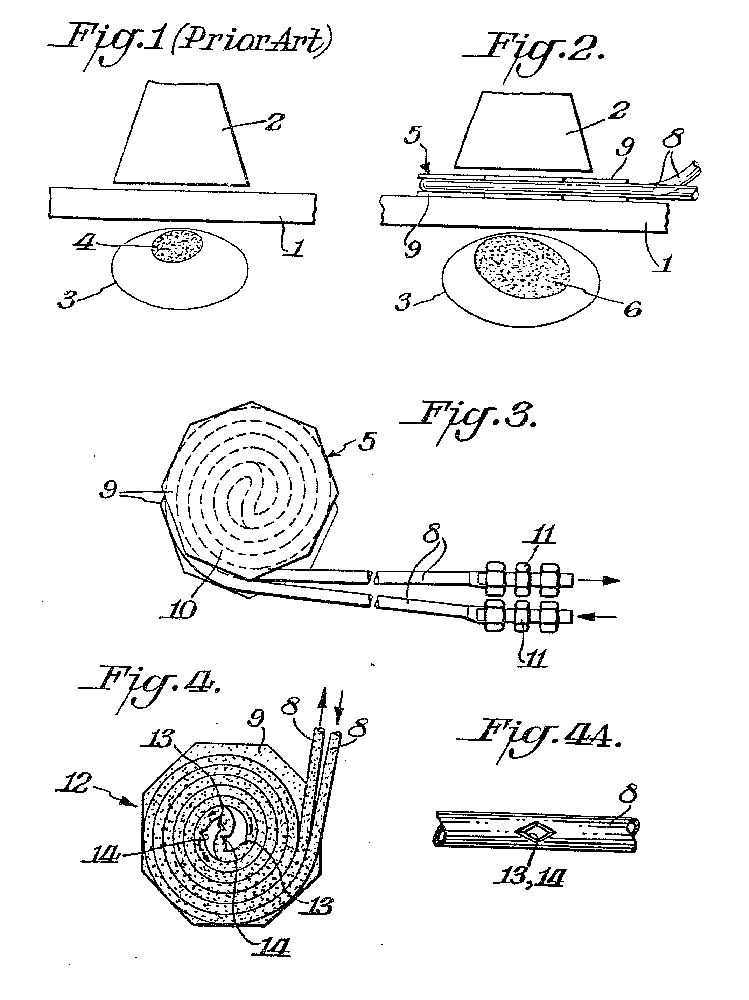

The prior art apparatus for the heat treatment of cancerous tumours as illustrated in Fig. 1 comprises a heater, such as a microwave antenna 2, which is disposed on epithelial tissue 1 to heat the cancerous region 4 of the cancerous organ 3 which may be located deep in the human body.

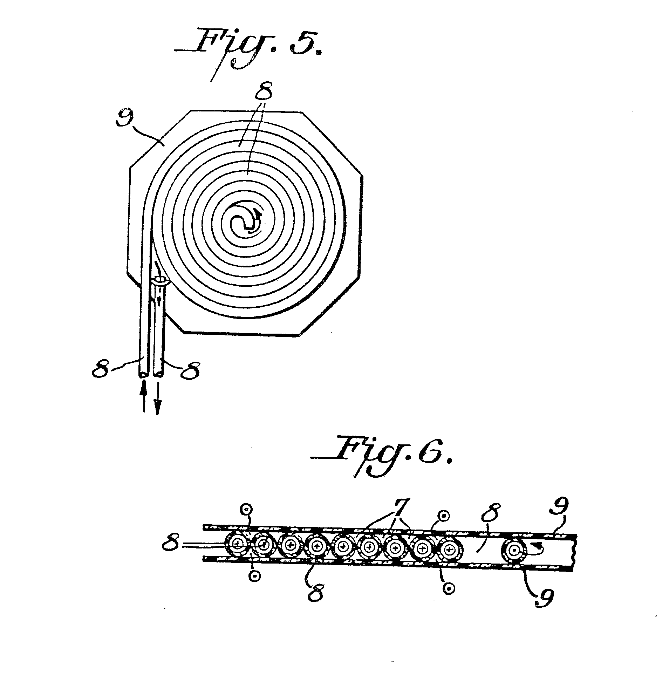

Referring to Fig. 2, the heat therapy apparatus according to the invention is seen to include a cooling device 5 . The device 5 is disposed between the epithelial tissue 1 and the microwave antenna 2 which acts as a heater. The device 5 is placed in direct contact with the tissue 1 and includes a tube 8 through which a refrigerant is caused to flow, the tube consisting of porous polytetrafluoroethylene having interconnecting pores. Accordingly, the cooling device is flexible and conforms to the epithelial tissue 1, and hence the device can effectively cool the tissue. Further, since the device 5 is made of inert polytetrafluorothylene it does not cause discomfort or physical disorder to the human body tissue 1.

In addition, since the portion of the cooling device 5 through which refrigerant is circulated has a great number of interconnecting pores, even if the refrigerant is warmed by the heater, the refrigerant is vaporized thereby and a large quantity of heat is removed, thereby cooling the tissue more effectively. Accordingly, even if a cancerous region 6 of the cancerous organ 3, or the like, is hypertrophied, the required heat energy can be given to the cancerous region while cooling noncancerous regions effectively with the device 5. Consequently, the cancerous region 6 can be treated effectively. Moreover, since the portion through which refrigerant is passed is made of polytetrafluoroethylene it has a small dielectric loss.  It follows that the tube is not heated by electromagnetic waves and that the heating efficiency is not lowered substantially.

Referring next to Fig. 3, an example of the cooling device 5 according to the invention is shown.  The device 5 comprises a tube 8 of porous, expanded polytetrafluoroethylene resin having interconnected pores.  The tube 8 is folded in two and wound into the form of a coil around the folded portion.  The two sides of the coil can be held in position by sheets 9 of a porous fluorocarbon resin having interconnecting pores, whereby a coiled portion 10 through which the refrigerant passes in contra-flow is formed.  Both ends  of the tube 8 are provided with couplings 11.

In the cooling  device 5 of Fig. 3, the sheets 9, holding the coiled portion 10, have interconnecting pores.  Therefore, if each of the sheets is in contact with the tube 8 at a point, the cooling due to the vaporization of refrigerant is not hindered.  Although the coiled portion is held by the sheets 9 on both sides, in the above embodiment, it is also possible to mount one sheet 9 on only one side of the coil.  The tube 8 and the sheets 9 can be fabricated by the process disclosed in Japanese Patent Publication No.

18991/1976, for example.

Fig. 4 is a fragmentary plan view of another cooling device according to the invention, the device being indicated by reference numeral 12. The cooling device is similar to the cooling device having the coiled portion 10 shown in Fig. 3 except in the following respects. The tube 8 and the sheets 9 are held in liquid-tight manner by leaving no space between the neighbouring turns, especially central turns, of the tube 8 made of porous, expanded polytetrafluoroethylene resin having interconnecting pores (contrary to the diagrammatic showing of Fig. 4). The coil is centrally provided with a refrigerant outlet port 13 and a refrigerant inlet port 14. Thus, in the cooling device 12 of this example, the cooling capability in the center is enhanced to some extent and can be preferably used together with a heater in which the heat energy density in the center is higher.

Other kinds of cooling devices can be fabricated using the same principles as in the cooling device 12 shown in Fig. 4. In particular, as shown in Figs. 5 and 6, a tube of porous polytetrafluoroethylene having interconnecting pores is first prepared. Then, the tube is wound into the form of a coil around one end of the tube. The top and bottom surfaces of the coil are joined and held in liquid-tight manner by sheets of a porous fluorocarbon resin having interconnecting pores. The tube is used as a refrigerant inlet tube. A passage 7 is formed between the outer boundaries of the tube and the inner surfaces of the sheets and has a substantially triangular cross section such that the refrigerant discharged from the inner end of the tube returns through this passage. In this case, it is required that the end of the return passage is provided with a refrigerant discharge tube as shown.

In the cooling device constructed as described just above, the return passage for the refrigerant has a triangular cross section and is provided between the neighbouring turns of the coil. Consequently, the velocity of the returning refrigerant is increased, and the area through which heat is dissipated is also enlarged. Further, the distance between the refrigerant and the object to be cooled is reduced, and the thermal resistance is decreased. In this manner, the cooling device can provide good cooling efficiency.

By means of the present invention it is possible to cool noncancerous regions efficiently without substantially reducing the heating efficiency of the heater, so that the required heat energy is provided to the cancerous region, thereby achieving improved treatment.

Another advantage is that it results in no physical disorder to the human body. Furthermore, because the portion through which the refrigerant is circulated has a number of interconnecting pores, it can provide a large cooling capabiliy by virtue of the heat of vaporization of the refrigerant, inspite of the presence of the heater.

Suitable refrigerants include water and alcohol. Refrigerants which are toxic or which could damage human tissue by contact would not be suitable. Bending between the porous tube and the porous sheets can be achieved by using suitable adhesives.

9  0194446

## CLAIMS

1. Apparatus for use in cooling the tissue surrounding a cancerous tumour in a patient undergoing heat treatment of the cancerous tumour by microwave energy characterised by at least one tube (8) wound in a flat coil of closely adjacent turns through which a refrigerant liquid can be caused to flow, the tube being made of a porous, expanded polytetrafluoroethylene resin having interconnecting pores.

2. Apparatus according to claim 1 characterised in that at least one side of the tube (8) is held in place by a sheet (9) of porous, expanded polytetrafluoroethylene having interconnecting pores.

3. Apparatus according to claim 1 characterised in that the coil comprises a coiled inlet portion and a coiled outlet portion disposed between the turns of the inlet portion.

4. Apparatus according to claim 1 characterised by a pair of sheets of a porous, expanded polytetrafluoroethylene resin having interconnecting pores, the sheets being joined to both sides of at least one coiled portion of the inlet tube in a liquid-tight manner to support said coiled portion.

Fig.1 (PriorArt)

Fig.2.

Fig.3.

Fig.4.

Fig.4A.

Fig. 5.

Fig. 6.

**European Patent Office**

**EUROPEAN SEARCH REPORT**

**0194446**

Application number

EP 86 10 1545

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | US-A-4 140 130 (STORM) <br> * claim 1; figures 10-14 * | 1 | A 61 F 7/10 <br> A 61 F 7/00 |
| Y | US-A-3 674 027 (FLEISCH MAJER) <br> * claim 1 * | 1 | |
| A | FR-A-1 072 012 (SCHOPFER) <br> * figures 2, 3 * | 1,2 | |
| A | DE-U-7 826 412 (EFFEN HAUSER) <br> * claims 1, 5, 6 * | 1,3 | |
| A | GB-A-1 448 068 (P. STEER DEVELOPMENTS LTD.) <br> * claims 1, 2; figures 1-3 * | 1,4 | |
| A | GB-A-1 366 631 (SPEMBLY TECHNICAL PRODUCTS LTD.) <br> * claims 1, 2; figure 1 * | 1 | TECHNICAL FIELDS SEARCHED (Int Cl 4) <br><br> A 61 F 7/00 |
| A | DE-C- 173 788 (BOEHM) <br> * claim * | 1 | |
| D,Y | WO-A-8 001 462 (BSD CORP.) <br> * claims 1, 4, 18 * | 1 | |

The present search report has been drawn up for all claims

| Place of search | Date of completion of the search | Examiner |
|---|---|---|
| BERLIN | 22-05-1986 | KANAL P K |